# EUROPEAN PATENT APPLICATION

(11) **EP 1 298 584 A2**
(43) Date of publication of application: **02.04.2003**
(21) Application number: 02021616.4
(22) Date of filing: 27.09.2002
(51) Int. Cl.: G06T 1/00

(54) **Method and apparatus to capture and analyze an image of a human body part**

(30) Priority: 01.10.2001 US 325559 P; 21.12.2001 US 24651
(71) Applicant: L'OREAL, 75008 Paris (FR)
(72) Inventor: Rubinstenn, Gilles, 75005 Paris (FR)
(74) Representative: Tanty, François

(57) **Abstract**

An image capture method and system are disclosed. The method includes instructing a subject to position a body part adjacent a display device and adjacent an image capture device. The method further involves sending a signal to the display device to generate light in a predetermined wave length range for irradiating the body part, and capturing an image of the body part with the image capture device while the body part is irradiated with the light, in the predetermined wave length range, emitted from the display device.

## Description

The invention relates to methods, combinations, apparatuses, and systems for conducting beauty analyses. The invention also relates to methods, combinations, apparatuses, and systems for capturing an image. In one example, the invention may integrate the use of image capture and on-line diagnostics.

Although the invention, in its broadest sense, is not limited to beauty products, this patent uses the beauty product example for purposes of conveying to the reader some of the principles of the invention.

Typically, highly personalized beauty attention is available through beauty facilities. Such facilities may include spas, salons, and specialized sales establishments. A benefit of using such facilities is that it allows professionals to test and view first-hand a subject's external body condition. Dermatologist, cosmetologist, skin/hair expert, or other reliable professionals have experience and/or quantitative tools to help a subject select appropriate beauty products.

With the advent of the Internet, has come a large number of portals selling beauty products and offering beauty advice. However, the advice tends to be generalized in the absence of hands-on testing and observation of the subject. Indeed, heretofor, personalized-quantitative analysis may not have been considered compatible with electronic channels of commerce.

Disclosed in this patent are various mechanisms for enhancing the quality of beauty assistance attainable at locations which may be remote from conventional testing and analysis facilities.

One exemplary aspect of the present invention may involve a method for facilitating analysis of an external body condition. The method may include offering to provide an analysis of at least one external body condition. A request for the analysis may then be received from a requestor. The requestor may then be provided with access to a computer program for transmitting an image of the external body condition. A transmitted image of the external body received from the requestor may be evaluated, and the requestor may be provided with information from the evaluation.

A second exemplary method of facilitating analysis may further include providing the requestor with a package for facilitating the analysis, the package including an image capture device for recording an image of the external body condition. The package also may include various testing materials.

An exemplary method of calibrating image capturing may include capturing a calibrating image of a body part adjacent a reference image using an image capture device. Calibration information may be generated by processing the calibrating image and information reflective of the reference image. The image capture device and/or a driver for the device may then be calibrated using the calibration information.

An exemplary method of facilitating capture of a body part image may include instructing a subject to position the subject's body part (e.g., face) adjacent an image capture device (e.g., in the field of capture of the image capture device). A first image of the subject's face may be captured using the image capture device and then processed. Processing may include calculating at least an approximate first position. Further, the calculated first position may be compared with a desired reference position, after which, the subject may be instructed to move the subject's body part closer to the desired reference position. A second image of the subject's body part may be captured after the subject has moved.

An exemplary method of providing a beauty analysis method may include instructing a subject to position a body part adjacent a display device and within a field of capture of an image capture device. A signal may be sent to the display device to generate light in a predetermined wave length range for irradiating the body part. An image of the body part may be captured with the image capture device while the body part is irradiated with the light emitted from the display device.

Another aspect relates to systems for performing at least portions of one or more of the methods.

A further aspect relates to a combination including at least one tool for gathering beauty related information, an image capture device and a driver for driving the image capture device.

The foregoing section is intended to provide the reader with a flavor of a few aspects of the invention. Therefore it is to be understood that both the foregoing description and the following detailed description are exemplary and explanatory only and are not restrictive of the invention as claimed.

The accompanying drawings, which are incorporated in and constitute a part of this specification, illustrate several aspects of the invention and, together with the description, serve to explain the principles of the invention. In the drawings,
Figure 1 illustrates a flow chart of an exemplary method for performing a beauty analysis consistent with the present invention;
Figure 2 illustrates another flow chart of an exemplary method for performing a beauty analysis consistent with the present invention;
Figure 3 illustrates a flow chart of an exemplary method for calibrating image capturing consistent with the present invention;
Figures 4a and 4b illustrate exemplary reference images for calibrating image capturing consistent with features and principles of the present invention;
Figure 4c illustrates an image of a body part being captured together with the reference image of Figure 4A, consistent with the invention.
Figure 5 illustrates another exemplary reference image for calibrating image capturing consistent with the present invention;
Figure 6 illustrates an exemplary method for processing an image of a body part consistent with features and principles of the present invention;
Figure 7 illustrates a flow chart of an exemplary method for facilitating capture of a body part image consistent with the present invention;
Figures 8A and 8B graphically illustrate aspects of an exemplary method for processing a subject's captured image consistent with the present invention;
Figure 9A and 9B graphically illustrate aspects of another method for processing a subject's captured image consistent with the present invention;
Figure 10 illustrates an exemplary system for comparing a position of the subject's image relative to a desired reference position consistent with the present invention;
Figure 11 illustrates a flow chart of an exemplary method for providing a beauty analysis consistent with the present invention;
Figure 12 illustrates an example of a combination in the form of a customized diagnostic kit consistent with features and principle of the present invention; and
Figure 13 illustrates an exemplary system environment consistent with features and principles of the present invention.

Reference is now made in detail to exemplary embodiments of the invention, examples of which are illustrated in the accompanying drawings. Wherever possible, the same reference numbers are used throughout the drawings to refer to the same or like parts.

One embodiment of the invention may include a method for facilitating analysis of an external body condition. Facilitating may occur, for example, by offering information or guidance to the customer so that the customer is encouraged to participate in an analysis of an external body condition. Such encouragement might also occur by providing a subject with a promotional item or a complementary service, as is discussed later in greater detail. An analysis might involve examination of body conditions such as skin texture, skin elasticity, skin or hair dryness, cellulitis, sweating, aging, wrinkles, melanoma, exfoliation, desquamation, homogeneity of skin or hair color, micro-circulation, skin or hair shininess, skin or hair softness, skin or hair smoothness, hydration, sebum production, skin or hair cleanliness, irritation, redness, vasomotion, vasodilation, vasoconstriction, pigmentation, freckles, baldness, thinning hair, size and proportion of facial features, or any other visible external body condition.

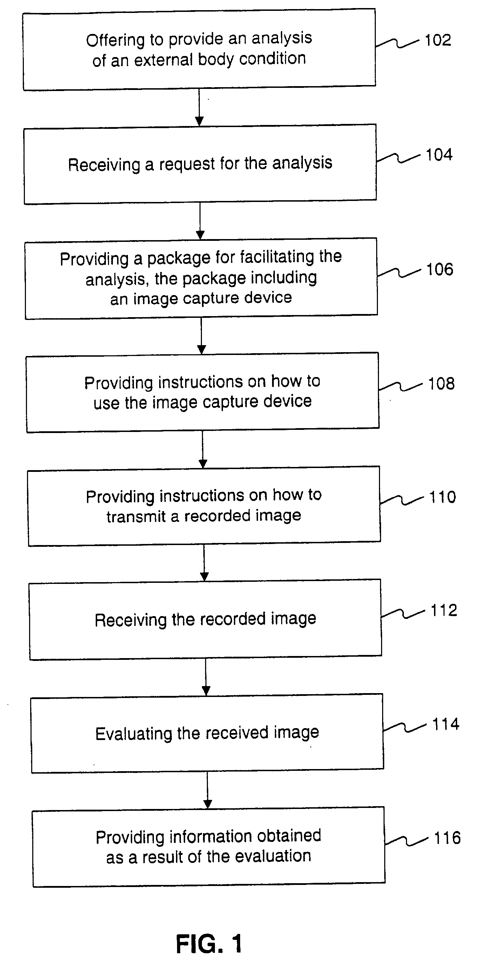

Consistent with the invention, a method may include offering to provide an analysis of at least one external body condition. This is shown as block 102 in the flow chart of Figure 1. The offer may be in written or oral form, and may include providing at least one of an express offer, an implied offer, a direct offer, and an indirect offer. The offer may be embodied in any type of information, promotion, or solicitation that refers to the analysis. Further, a first party may operate alone or in cooperation with a third party vendor to provide the offer. For example, text on a web page might directly invite a subject to seek an analysis from the web hoster. Or similar text may indirectly offer an analysis to be performed by a third party.

Offers may be made through electronic advertisements or printed media. Electronic advertisements may be transmitted by e-mail or facsimile over a network, transmitted physically or electronically by software configured to run on the recipient's computer, or embedded in various websites, including pop-up advertisements or Internet links that direct an individual to a third party website. Printed media containing an offer may be distributed by mail, facsimile, flyers, magazines, or simply displayed as advertisements within or outside a store. In addition, sales representatives may discuss the offer face-to-face with potential customers, or telemarketers or automated telephone dialing services may call potential customers to discuss the offer or provide pre-recorded voice message advertisements.

Consistent with the invention, a method may include receiving a request for the analysis, as graphically depicted at block 104 in Figure 1. The term "receiving" as used throughout this patent may include at least one of receiving a request over a network, receiving a request verbally, receiving a request electronically, receiving a request telephonically, receiving a request in hard-copy form, or receiving a request through any other mechanism enabling reception. In addition, "receiving" may occur either directly or indirectly. For example, receipt may occur through a third party acting on another party's behalf, as an agent of another, or in concert with another. Regardless, all such indirect and direct actions are intended to be covered by the term "receiving" as used herein.

The received request may take one of many forms. It may be written, oral, or electronic. It may simply be a checked box, clicked button, submitted form or oral affirmation. Or it might be a typed or handwritten textual request.

Receiving may occur through an on-line interest form, e-mail, facsimile, telephone, interactive voice response system, or file transfer protocol transmitted electronically over a network at a website, an internet protocol address, or a network account. A request may be received from a subject for whom the analysis is to be conducted, or an entity acting on the subject's behalf.

A network used with some embodiments of the invention may include local area networks, wide area networks, metropolitan area networks, or any other type of network suitable for interfacing a host website with the subject's workstation. The network may include one or more of wired and wireless connections. The network may also include a combination of public (e.g., Internet) and private networks, as well as virtual private networks. In a broader sense, a network may include any mechanism for facilitating communication between two nodes or remote locations. One of ordinary skill in the art can readily appreciate that any number of geographically dispersed workstations may connect to the network and couple with a host website.

Printed media may be received in hard-copy form. Similarly, a subject may respond orally to an offer during a telephone call. The requestor may talk to an operator, press a keypad on the telephone in response to prompts, or leave a voice message to request the analysis. In addition, the subject may accept a face-to-face offer from a sales representative. The received request may include an indication of acceptance of the offer, as well as other personal information, such as a name and mailing address of a subject or the requestor.

Consistent with the invention, a method may include providing a package for facilitating the analysis, the package including an image capture device for recording an image of the external body condition. This is graphically illustrated at block 106 in Figure 1. Facilitating may occur by virtue of the fact that material contained in the package is to be used for carrying out at least a portion of the analysis. To this end, and according to one aspect of the invention, the only substantive item contained in the package may be an image capture device.

"Providing," as used throughout this patent, includes direct and indirect activities. Thus, an entity who hires, works with, or cooperates with another to convey a package, performs the act of providing, despite the indirect nature of the entity's conduct. As used herein, an "image capture device" may include a web camera, an analog camera, a digital camera, a flat bed scanner, a film scanner, ultra-sound imaging device or any other device suitable for capturing an image of the subject. The package may be provided to the subject by courier or any other package delivery service. A first party may operate alone or in cooperation with a third party vendor to provide the requestor with the package. The package may also include a driver for driving the image capture device.

Consistent with the invention, the image capture device may be provided to the requestor free of charge. In this manner, the image capture device may serve as an interactive marketing tool for engaging the subject. Alternatively, the package may be offered at a fair market value or at a discounted value.

While the image capture device may be usable by the subject for a variety of purposes, at least one intended purpose in connection with the invention is to capture an image of an external body condition. To this end, the package might include instructions to the user on how to perform body image capture. The instructions might be in hard copy or electronic form, and may be transmitted with or separated from the image capture device.

The package also may (or may not) include an evaluation tool for analyzing an external body condition. The evaluation tool may include at least one of a pH indicator, a sebutape, a d-squame disc and a corneodisque indicator. The subject may use a pH indicator, according to the electronic or hard-copy instructions, to test a pH value of the skin and/or scalp. Similarly, the subject may use sebutape to remove sebum and other liquids, such as lipids, from the surface of the skin or scalp, thereby indicating skin oiliness, and a d-squame discs to remove flakes from the surface of the skin or scalp, thereby indicating skin dryness. A corneodisque indicator may leech water from the surface of the epidermis, thereby indicating the moisture/humidity content of at least the epidermis portion of the skin.

Other tests of varying cost and scope may also be provided in the package. For example, test materials may include a device to measure skin elasticity. Similarly, test materials may include hormone tests, enzymatic tests, or any other measurement reflective of an external body condition. Test materials may also include various color charts (in hard or soft form), which a subject may use to compare with an external body condition. For example, the subject may use such charts to determine a skin or hair tone, skin or hair color, amount and intensity of wrinkles, acne, balding, hairiness, and/or freckles by selecting the chart that most closely represents an external condition of the subject. Skin roughness may be may be judged using tactile test materials as a reference against which skin texture may be determined. Similar tactile test materials may be used for hair and skin texture and color. The comparison may be completed after applying a particular product to the skin to increase effectiveness. In addition, test materials may include materials for the subject to compare with a texture of the skin to determine a roughness level of the skin.

If the package includes multiple items, all items may be transmitted as a group. Alternatively, the package may include multiple parcels transmitted at the same or separate times. The package may include, for example, materials received by the subject in person at a retail establishment and materials later received by courier.

The test materials may include a voucher for having one or more tests conducted at a testing facility. Directions may be provided on how to conduct a self-test (either alone or with the aid of an acquaintance) and may further include instructions on how to interpret results of the self-test. Alternatively, the directions might include information on how to go about transmitting used self-testing materials to a vendor for analysis. To that end, a voucher or other authorization mechanism may be provided as part of the package, for transmission to the vendor.

In lieu of one or more self-testing materials, the included voucher or authorizing mechanism might cover a face-to-face analysis in a testing facility. In that case, the subject might be advised of a nearby testing facility where the voucher may be redeemed in person. The testing facility may use at least one of a corneometer for measuring a water content of subject's scalp or skin, a cutometer for measuring skin elasticity, a melameter for measuring a melanin index and hemoglobin (erythema) index of skin, a sebumeter for measuring sebum content of scalp or skin, skin-pH meter for measuring pH of skin or scalp, skin visiometer for measuring skin roughness, sun protection diagnostic to perform an SPF analysis, tewameter to measure trans-epidermal water loss, visioscan to evaluate the skin surface, chromameter to measure skin clarity or luminosity, impedance measurements to measure skin moisture, a gas bearing electrodynamometer to measure skin softness and suppleness, skin replicas or image analysis to measure skin surface smoothness or texture, TEWL to measure skin barrier function, laser doppler to measure blood flow or skin sensitivity, ultrasound to measure skin and fatty tissue thickness, lesion counting and bacteriology to study acne, and ballestometry to measure skin firmness. Hair moisture and suppleness, hair tensile strength, penetration of actives into hair, hair deposition, and UV protection may also be determined. The tests and test materials described above are not intended to be inclusive, it being understood that tests and test materials, as used herein, are intended to cover any method and product for evaluating an external body condition.

Consistent with the invention, a method may include providing instructions on how to use the image capture device to record an image of the external body condition, as graphically shown at block 108 in Figure 1. The instructions may be provided electronically, telephonically, verbally, via software or in hard copy form. Further, they may be provided directly or indirectly as discussed earlier. The instructions may be provided electronically at a website, which the subject may access over a network, or they may be transmitted over a network electronically through e-mail or by file transfer protocol. In addition, the subject may receive software configured to run on the subject's computer for providing the instructions electronically.

The instructions may include information on how to connect the image capture device to a computer. For example, the instructions may explain how to connect the image capture device to at least one of a universal serial bus (USB), a SCSI card for a PCI or ASP port, another dedicated card, and a parallel port of a computer. The instructions may also contain information regarding when, where, and how to use the image capture device. For example, the instructions may suggest using the image capture device at a particular time of the day for lighting purposes, or in a darkened room with a particular light source. The instructions might also suggest use of the image capture device only after a prescribed amount of time following cleansing or application of certain other beauty products. For example, the instruction may suggest waiting several hours after cleansing and/or applying any beauty products before using the image capture device. In addition, the instructions may direct where to stand in relation to the camera and how to position the image capture device.

Consistent with the invention, a method may include providing instructions on how to transmit a recorded image of the external body condition to an evaluation location. This is graphically illustrated at block 110 in Figure 1. These instructions may be transmitted in a manner similar to that, described previously, in connection with instructions for using the image capture device. The instructions may direct the user on how to transmit an image over a network such as the Internet. Alternatively, the instructions may direct the user to save the recorded image on a storage medium and physically transmit the storage medium to a designated location. Examples of storage medium may include magnetic storage devices, such as floppy disks, lomega zip disks, and tapes, optical storage devices, such as compact discs and digital video discs, organic storage devices, random access memory, read only memory, printed media, or any other medium for storing information. The instruction might also direct the user to print the recorded image and send the printed image by mail or facsimile to a designated location.

By way of yet another example, the user may be instructed to provide the recorded image electronically to a client-based algorithm or a server-based algorithm. The client-based algorithm may be provided on a storage medium configured to run on the recipient's computer after delivery to the recipient. A server-based algorithm may be provided at a location remote from the recipient and/or through a third party vendor. The recipient may provide the algorithm to the server based algorithm over a network. One of ordinary skill in the art will appreciate that a first party may operate alone or in cooperation with a third party vendor to instruct the user on how to transmit a recorded image.

Consistent with the invention, a method may include receiving the recorded image at the evaluation location, as illustrated in block 112 of Figure 1. The recorded image may be received electronically, telephonically, in software form, or in hard-copy form as described above. Electronic reception may occur at a node on a network. Physical reception may occur at a facility associated with persons skilled in visually or electronically evaluating images of external body conditions. For example, the technician may note shininess depicted in an image of the external body condition, indicating an oily skin condition or a technician may note the intensity and severity of facial wrinkles. And as discussed previously, receiving may occur directly or indirectly.

Consistent with the invention, a method may include evaluating the received image. This is graphically illustrated in block 114 of Figure 1. The term "evaluating" may include, but is not limited to, one or more of observing, marking, following, recording, graphing, measuring, calculating, projecting, and monitoring the image. For example, evaluating may include generating metrics of desirable and undesirable conditions in the subject's external body condition. Examples of metrics for wrinkles may include average density, extent, intensity, frequency, type and severity (e.g., calculated from wrinkle depths and percentage area). Examples of metrics for shininess may include extent, intensity, frequency, type, and severity of shininess (e.g., calculated from reflectivity indices).

Image processing techniques and/or software may be used to evaluate the external body condition. Techniques for processing images may involve binarizing the received image to aid in locating conditions. Binarization may increase the contrast of the image to facilitate condition detection. Fourier transforms, fast Fourier transforms (FFTs), and/or discrete cosine transforms may be performed on all or part of the image, resulting in coefficients. Based on the coefficients, conditions may be located, as known in the art. Artificial intelligence, such as fuzzy logic, neural networks, genetic programming and decision tree programming, may also be used to identify conditions. It is known to persons of ordinary skill in the art how to train a neural network to accomplish these functions. Alternatively, one or more digital filters may be passed through the image for locating specific conditions. These examples are provided for illustrative purposes with the understanding that any image processing technique may be used within the scope and spirit of the invention.

Also consistent with the invention, a technician or other skilled individual may visually examine the image.

As with the other steps, "evaluating" may be accomplished directly or indirectly. An example of indirect evaluating includes partnering with a third party who performs the evaluating function.

Consistent with the invention, a method may include providing information obtained as a result of evaluating the received image. This is graphically depicted in block 116 of Figure 1. "Providing" may occur using mechanisms previously described to convey to a subject information learned as a result of evaluating. For example, the subject may be advised that she has unusually severe wrinkles for her age. The provided information may also advise whether a treatment exists for the condition. Further, the information may include identification of a recommended product. For example, the method may provide an individual with advice regardless of form, suggesting either directly or indirectly to use one or more beauty products (e.g., cosmetic substances and/or cosmetic services). Such a suggestion may occur in response to a specific request from the individual, or may be made in response to collecting individual-specific information provided through electronic or physical channels. The prescription may suggest use of a specific product(s), class of product(s), and/or a product brand. In addition to product recommendations, the prescription may include preventative measures, remedial measures, lifestyle or dietary recommendations or any advice or guidance that might correlate to a subject. The prescription may or may not contain product application and use advice.

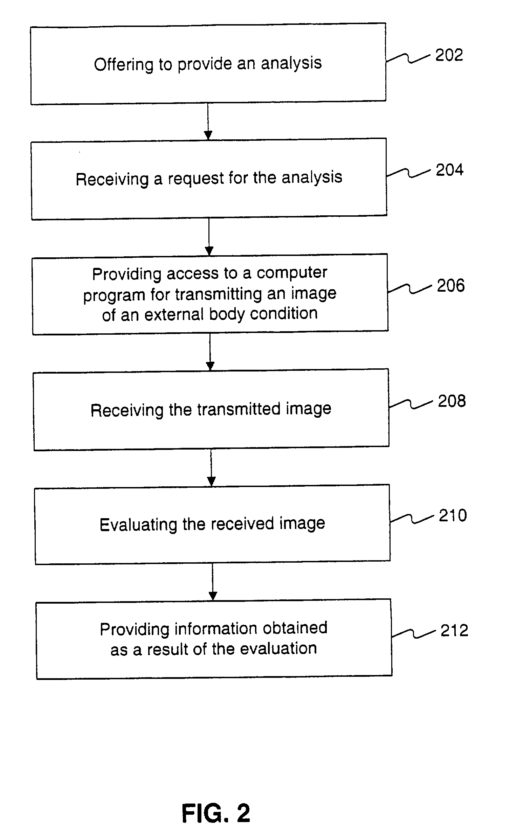

Another embodiment of the invention, graphically illustrated in a flow chart of Figure 2, also depicts a method of facilitating analysis of an external body condition. This embodiment is similar in many respects to the embodiment of Figure 1. However, rather than providing a package including an image capture device (block 106 of Figure 1) and instructing the requestor on how to use the image capture device (block 108 of Figure 1), the embodiment of Figure 2 may include providing access to a computer program for transmitting an image of an external body condition (block 206 of Figure 2.) Block 206 of Figure 2 may be performed after offering to provide an analysis of at least one external body condition (block 202, which is similar to block 102 of Figure 1) and after receiving a request for the analysis (block 204, which is similar to block 104 of Figure 1.)

Providing access to a computer program may include at least one of providing access over a network to the computer program, and creating or distributing to the subject a computer program configured to run on the subject's workstation or computer. For example, a first party may host a website or contract with a third party vendor to host a website for enabling a user to access the computer program over a network. The user may receive the computer program by downloading it from a host website, by having it delivered via file transfer protocol (FTP), to a workstation local to the recipient, by receiving it as an attachment in an e-mail message, or any other mechanism for obtaining a computer program. The computer program may be provided on software, hardware, or a combination thereof.

Access to the computer program may be provided to the subject of the analysis. Alternatively, access may be provided to an entity acting on the subject's behalf.

Alternatively, a first party and/or third party vendor may enable system access by creating or distributing to the subject a floppy disk, tape, compact disc, digital video disc, organic storage device, lomega zip disk, or any other storage medium containing a computer program configured to run on the recipient's workstation. The recipient's workstation may be embodied within a home computing system, may be located in a kiosk or mobile communications device, or may be part of a processing system run by professionals in a salon, place of business or retail establishment. As embodied herein, the computer program configured to run on subject's computer may be delivered to the subject using a courier, Federal Express, United Parcel Service, United States Postal Service, or any other public or private delivery service.

Consistent with the invention, a method also may (or may not) include receiving the transmitted image of the external body condition (block 208), evaluating the received image (block 210), and providing information obtained as a result of evaluation (block 212). Blocks 208, 210, and 212, which are graphically depicted in Figure 2, are similar to blocks 112, 114, and 116, respectively, of Figure 1.

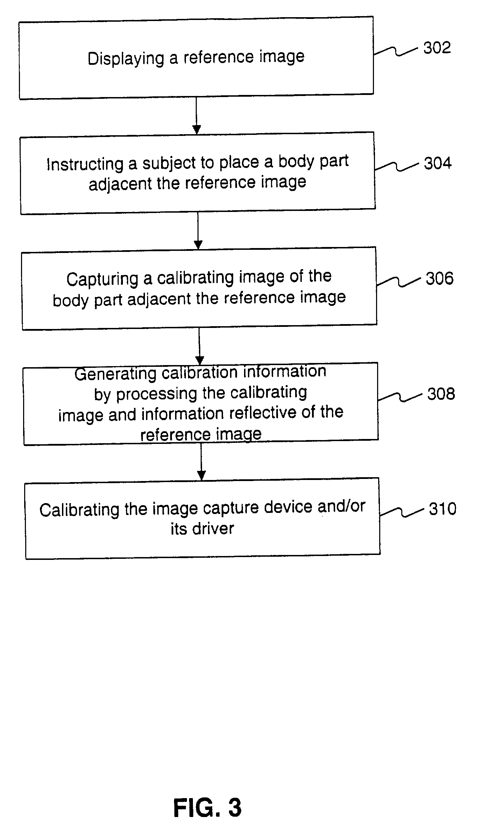

As illustrated in a flow chart of Figure 3, another embodiment of the invention may also include a method of calibrating image capturing. Consistent with the invention, the method may (or may not) include displaying a reference image on a calibrated display device. This is illustrated in block 302. "Displaying" may include providing access to at least one of a client-based algorithm and a server-based algorithm for displaying the reference image. In addition, as used herein, "displaying" includes a direct act of causing display, as well as any indirect act that facilitates displaying. Indirect acts include providing software to an end user, maintaining a website through which a user is enabled to affect a display, hyperlinking to such a website, or cooperating or partnering with an entity who performs such direct or indirect acts. Thus, a first party may operate alone or in cooperation with a third party vendor to enable the reference signal to be generated on a display device.

The display device may include any device suitable for displaying the reference image, such as a monitor. In one example, the display device may be a device which has been calibrated through the use of any conventional software intended to be used in evaluating, correcting, and/or improving display results (e.g., a color monitor that has been adjusted using monitor calibration software).

Such calibration techniques are employed, for example, by Hewlett Packard and Lexmark printer drivers which account for a transfer from a RGB display mode to a CMYK print mode.

Alternatively (or in addition), a special lighting device (for example, a special lighting device located at a salon) could be used to obtain information that could then be used to calibrate a display.

If the display device has been calibrated, precise colors of a reference image displayed on the display device may be known. If a body part is placed adjacent the known reference image, an image capture device might be used to simultaneously capture an image of both the body part and the reference image. A color match might be then be determined. Thereafter, the image capture device (or its driver) might be calibrated.

Rather than (or in addition to) displaying the reference image on a display device, a method, consistent with the invention, may include providing a reference image to a subject. "Providing" may include creating or distributing the reference image to the subject by physical, telephonic, or electronic delivery, providing access over a network to the reference, or creating or distributing software to the subject configured to run on the subject's workstation or computer including the reference image. In one example, the providing of the reference image could involve enabling the subject to obtain the reference image in hard copy form via a printer. For example, information, software, and/or instructions could be transmitted (e.g., electronically or physically via a data storage device or hard copy) and/or otherwise made available (e.g., via a network) in order to facilitate the subject using a printer to print a hard copy form of reference image. In such an example, the printer may be a printer which has been calibrated through the use of any conventional software intended to be used in evaluating, correcting, and/or improving printing results (e.g., a color printer that has been adjusted using color correction software).

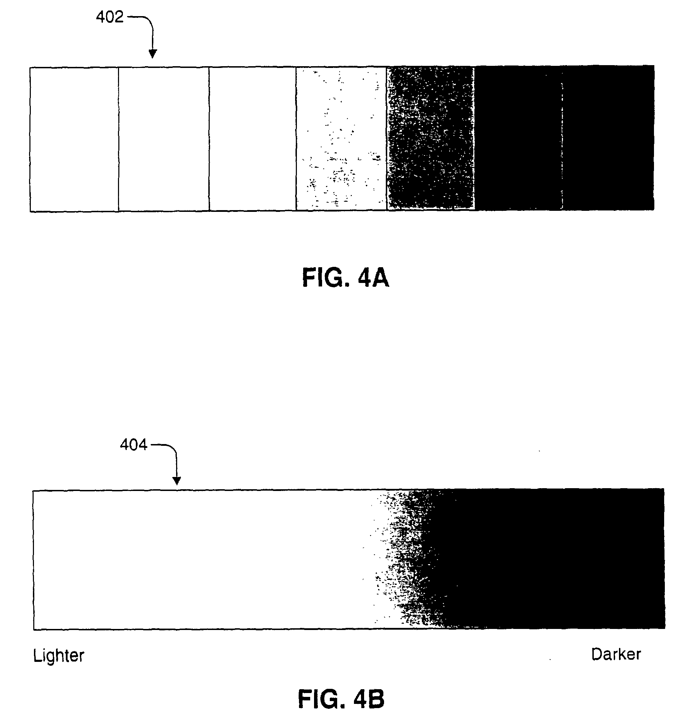

The reference image may include a color bar of multiple colors and/or a swatch of multiple colors. The multiple colors may represent a range of skin tones, skin colors, and/or skin replicas. For example, a range of skin tones or skin colors may include a monotonic range of discrete or continuous tones or colors. Figures 4a and 4b graphically illustrate a discrete and a continuous range of colors, respectively (depicted in gray scale).

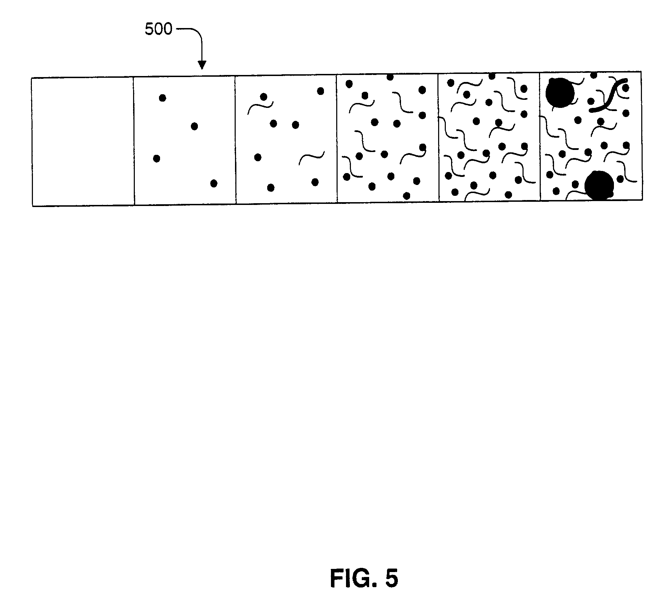

Alternatively, a skin replica may include a range of continuous or discrete skin replicas or patterns, each skin pattern including at least one color. Skin replicas, in physical form, may include a synthetic swatch that might be included, for example, in a test kit. In image form skin replicas may include actual or synthesized images of skin. The skin replica or pattern may indicate a predominate color or tone of an area of skin, as well as a degree of variation in the skin color or tone. Particularly, the skin replica may indicate a level of freckles, moles, hair, age spots, blotches, or other skin irregularities. An exemplary skin replica having discrete skin patterns for one predominate color is shown in Figure 5. One of ordinary skill in the art can readily recognize that, within the scope and spirit of the invention, skin patterns may be provided in numerous forms and for numerous tones and colors of skins.

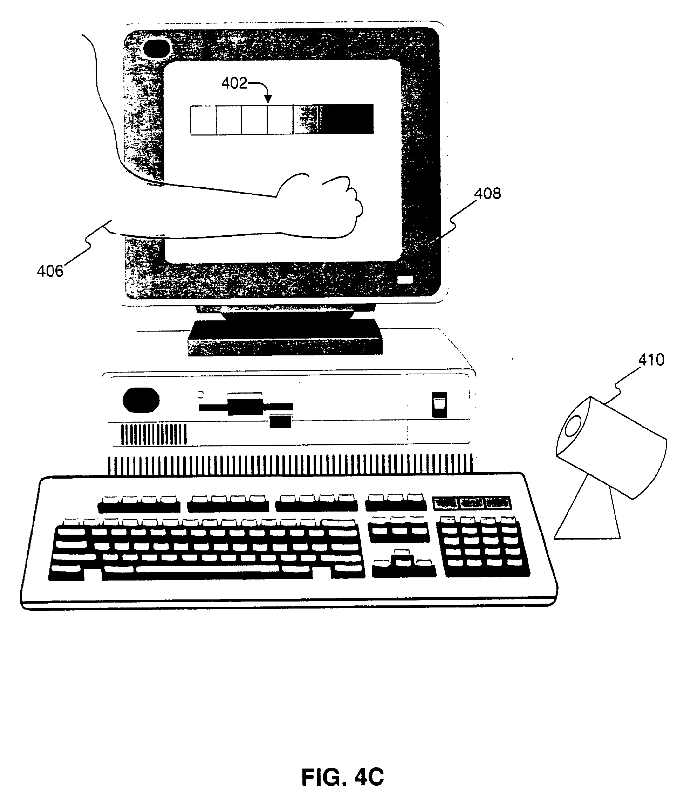

Consistent with the invention, a method may include instructing a subject to place a body part adjacent the reference image. This is graphically illustrated at block 304 of Figure 3. For example, the subject may be instructed to place an arm, hand, face, or other body part adjacent the reference image displayed on the display device, as shown in Figure 4c. Figure 4c graphically illustrates capturing an image of an arm 406 placed adjacent a reference image 402 displayed on display device 408 using web cam 410.

The instructions may further advise the subject to position the body part such that the displayed reference image and the body part may be captured simultaneously by an image capture device. Thus, the term "adjacent" includes all positions where the body part and the reference image may be captured together by an image capture device. The phrase "instructing the subject" may include at least one of transmitting instructions to the subject over a network, transmitting instructions to the subject in the form of software, and instructing the subject in hard-copy form as detailed above. It also includes providing instructions to someone acting on the subject's behalf. A first party may operate alone or in cooperation with a third party vendor to instruct the subject.

Consistent with the invention, a method may include capturing a calibrating image of the body part adjacent the reference image using an image capture device. This is depicted at block 306 of Figure 3. As used herein. the "capturing" could be performed directly (e.g., by operating the image capture device) or indirectly (e.g., by enabling and/or facilitating operation of an image capture device to obtain the calibrating image). For example. capturing may be performed by providing instructions on how to use an image capture device, as discussed above. Capturing a calibrating image may include providing access to at least one of a client-based algorithm and a server-based algorithm for causing the image capture device to capture a calibrating image. Capturing may also include at least one of receiving an instruction from the subject to capture a facial image, indicating to the subject that the facial image is about to be captured, and indicating to the subject when a facial image is captured. In one example, the reference image and/or the body part may be spaced away from the image capture device when the calibrating image is acquired (e.g., the reference image and/or body part could lack contact with and/or physical connection to the image capture device used to acquire the calibrating image).

In one optional example, where the reference is displayed on the display device, the display device may be darkened except for the area of the reference image, so that excess light from the screen does not interfere with image capture calibration. Alternatively, the lighting in the portion of the screen other than the reference image could be adjusted to illuminate the body part during image capture.

Consistent with the invention, a method may include generating calibration information by processing the calibrating image and information reflective of the reference image, as shown at block 308 of Figure 3. The phrase "generating calibration information" may include performing any type of image processing or analysis on the calibrating image and information relating to the reference image, which may then be used for creating and/or storing information to calibrate the image capture device and/or a driver for the image capture device (e.g, software, a processor, and/or any form of hardware associated with the image capture device to enable its operation). The generating of calibration information could be performed via direct activity (e.g., conducting the calibration generation) or indirect activity (e.g., transmitting and/or making available software, an algorithm and/or information enabling and/or facilitating calibration information generation). The "information reflective of the reference image" may be the reference image itself and/or data regarding the reference image (e.g., information indicative of the actual colors and/or color distribution in the reference image).

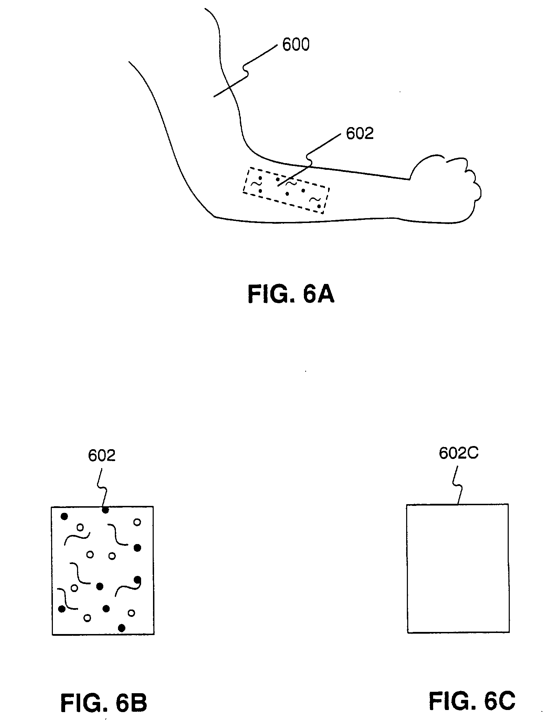

Generating calibration information may also include comparing at least one color of the body part in the calibrating image to at least one known color of the reference image. For example, when the reference image comprises a monotonic range of skin tones or skin colors, as illustrated in Figures 4a and 4b, the calibrating image may be filtered or otherwise processed for determining a predominate color prior to the comparison. For example, Figure 6a depicts an image of an arm captured as a calibration image. At least a portion of the captured image 602, graphically shown in an enlarged view in Figure 6b, may be processed using a digital filter or other processing technique, for example, to remove moles, freckles, age spots, hair, and other irregularities from the image, thereby determining a predominate color or tone 602c, shown in Figure 6c.

After determining the predominate color or tone, the filtered calibration image may be compared to at least one known color of the reference image. For example, the filtered image 602c shown in Figure 6c may be compared to at least one color of the reference image 402, 404, shown in Figures 4a and 4b, respectively. Based on the comparison, a color of the reference image that is most similar to a color of the calibration image 602c may be stored as calibration information in a data base provided at a location local or remote from the subject. Since the colors on the color bar are known quantities, a single image of the color bar and the skin will enable precise calibration of the image capture device. Alternatively, if a color bar or skin replica bar is provided to the subject in physical form, the image capture device might capture an image of the color bar on or near the skin within the same image.

When the reference image comprises a range of skin replicas, the calibrating image may be compared to a reference image, such as reference image 500 of Figure 5, without filtering. In this manner, a skin replica most similar to the original calibrating image 602 may be automatically selected and stored in a data base as calibration information.

Consistent with the invention, a method may include calibrating the image capture device and/or the driver for the image capture device using the calibration information. This is graphically shown at block 310 of Figure 3. "Calibrating" may include enabling an adjustment on a subsequent captured image based upon the calibration information. For example, a color, tone, or skin replica/pattern of a subsequent captured image may be adjusted to resemble a color, tone, or skin replica/pattern that is stored as calibration information. In this way, an accurate image of the subject's external body condition may be displayed on the display device, even under varying lighting conditions, for example. As with other aspects of the methods described herein, the calibrating could involve direct activity or indirect activity (e.g., providing and/or making available an algorithm, software and/or information enabling and/or facilitating the calibrating).

Although not required in its broadest sense, a method consistent with the present invention may further include capturing an image using the calibrated image capture device and/or driver and providing a beauty analysis based upon the captured image. The beauty analysis may provide information regarding an external body condition, as described above.

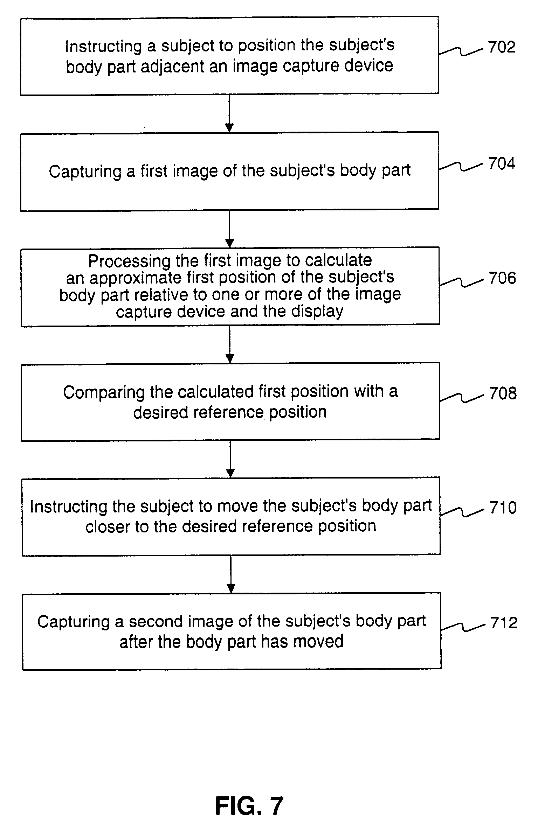

As illustrated in the flow chart of Figure 7, another embodiment of the invention may include a method for facilitating a capture of a body part image. Consistent with the invention, a method may include instructing a subject to position the subject's body part within a field of capture of an image capture device. This is graphically illustrated at block 702 of Figure 7. Instructing the subject may include providing access to at least one of a client-based algorithm or a server-based algorithm for directing the subject. Instructing may be accomplished directly or indirectly, using mechanisms previously disclosed. In one example, the "body part" could be the subject's face, but the method is not limited to facial imaging. As used herein, "field of capture" is intended to include the area adjacent to an image capture device where the device is capable of capturing an image. For example, for a web cam, the field of capture could relate to the area within the focal view of the web cam.

Consistent with the invention, a method may include capturing a first image of the subject's body part using the image capture device, as depicted at block 704 of Figure 7. "Capturing" a first image may occur directly or indirectly and include providing access to at least one of a client-based algorithm and a server-based algorithm for causing the image capture device to capture a first image. Also consistent with the invention, capturing may include at least one of receiving an instruction from the subject to capture a body part image, indicating to the subject before a body part image is captured, and indicating to the subject when a body part image is captured. "Capturing" may also include providing software to a user so that a user may be aided in the capture process.

Consistent with the invention, a method may include processing the first image to thereby calculate at least an approximate first position of the subject's body part relative to one or more of the image capture device and a display device (e.g., a display device of which an image of the body part is intended to be displayed). This is shown at block 706 of Figure 7. The phrase "processing of the first image of the subject's body part" may include at least one of determining whether the first image includes an entire image of the subject's body part, determining whether the first image includes a portion of the subject's body part, determining whether the subject's body part is centered in the first image, and determining a size of the subject's body part in the first image. The processing may be performed using artificial intelligence, including neural networks and fuzzy logic. The processing may be accomplished directly or indirectly, using any mechanism previously disclosed.

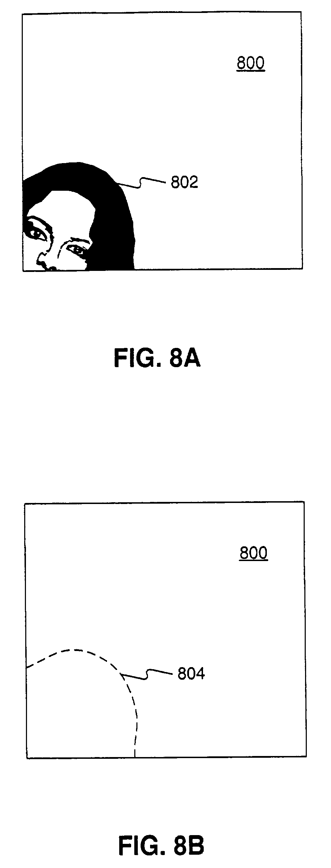

Image processing techniques may be employed to extract and analyze facial features of the captured image, thereby determining whether an entire image of the subject's face has been captured. For example, a computer program may extract an outline of a human head and analyze the outline to determine whether an entire image of the subject's face has been captured, as illustrated in Figures 8a and 8b. Figure 8a depicts an image of a portion of the subject's face 802 . An outline of the image 804 may be extracted, as shown in Figure 8b. Upon analysis of the image using known image processing techniques, such as pattern matching, for example, it becomes clear that the outline 804 does not resemble the complete outline of a human head.

Similarly, a computer program may extract and analyze other features of the subject's body part to determine whether an image of the entire face has been captured. For example, the computer program may determine whether the image includes at least one of a pair of eyes, a mouth, a chin, and a forehead. In Figure 8a, image processing techniques may reveal the image 802 does not include a mouth or a chin. Thus, the computer program may conclude that the captured image depicts only a portion of the subject's body part, and the computer program may evaluate a position of the subject relative to the image capture device based on the partial image. For example, in Figure 8a, the positioning of the subject's face 802 indicates that the subject, from the subject's view point, is to the right and slightly below the focal plane of the image processing device.

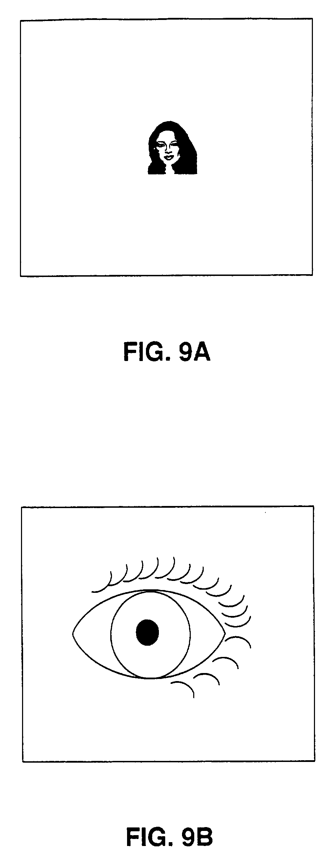

Similarly, image processing techniques may be employed to measure a size of the subject's body part in the captured image. By evaluating a size of the subject's body part in the first image, the computer program may determine an approximate first position of the subject's body part relative to the image capture device. For example, if the subject is positioned too far from the image capture device, the subject's facial image might comprise a small area within the first image, as shown in Figure 9a. Further, if the subject is positioned too close to the image capture device, a portion of the subject's facial image may comprise a large area or an entire area of the captured image, as shown in Figure 9b.

Consistent with the invention, the phrase "processing of the first image" also may include determining whether the first image is properly illuminated. By determining whether the first image is properly illuminated, the computer program may determine an approximate first position of the subject's body part relative to the display device. For example, if upon processing, the first image of the subject's body part appears dark, the image processor may instruct the subject to move closer to the display device for illumination. Alternatively, if the image is dark, the aperture of the image capture device may open to allow more light to pass through. As with other aspects of the methods described herein, the processing could involve direct or indirect activity.

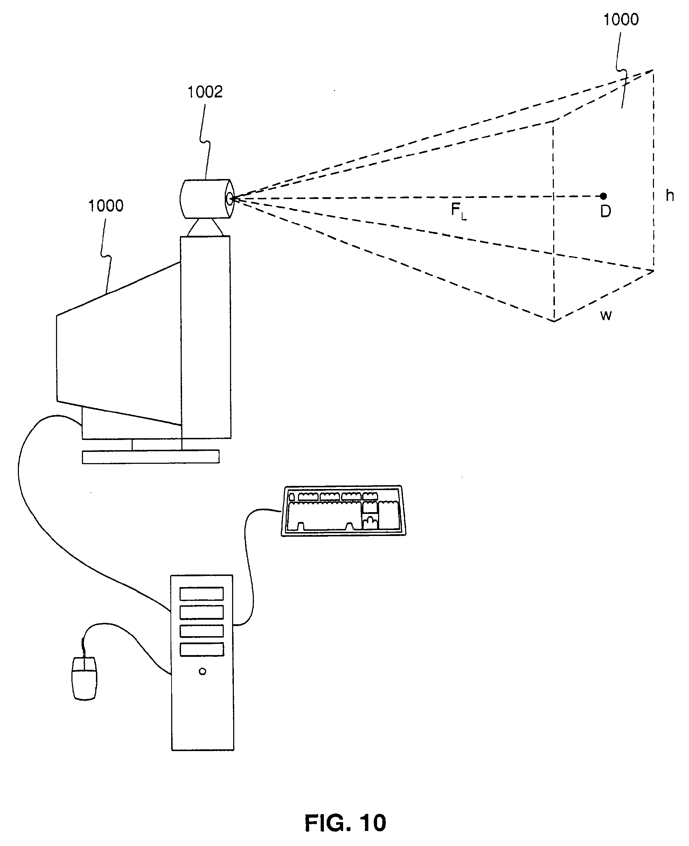

Consistent with the invention, a method may include comparing the calculated first position with a desired reference position, as graphically illustrated at block 708 in Figure 7. As shown in Figure 10, a desired reference position may include a position D, which is a center of a focal window 1000 located at a focal length FL of an image capture device 1002. The focal length FL may include a distance at which the image capture device 1002 may focus on an object, and a focal window 1000 may include an area, having a width w and a height h, captured by the image capture device at the focal length. The computer program may approximate the distance the subject needs to move in relation to a known focal length of the image capture device and a known size of the focal window. For example, in Figure 8a, the computer program may approximate the distance the subject must move to position a center of the subject's facial image at the center of the focus window 800. One of ordinary skill in the art will readily recognize that the focal length and focal window may comprise a range of values. For example, a image capture may have a zoom lens, providing an additional focal length and focal window area. Here again, the comparison could involve either direct or indirect activity, as described earlier.

Consistent with the invention, a method may include instructing the subject to move the subject's body part closer to the desired reference position, as graphically illustrated at block 710 of Figure 7. Instructing may include instructing the subject about how to move (e.g., the direction and/or amount of movement for moving closer to the desired position). Instructing the subject may include directing the subject to move when the displacement is greater than the threshold value. In this manner, the first image may be accepted and processed if the displacement between the approximate first position of the subject's body part and the desired reference position is within an acceptable range. "Instructing the subject on how to move" may include providing access to at least one of a client-based and a server based algorithm for instructing the subject to move. Instruction may be provided in the form of text, or in the form of symbols (e.g., arrows depicting direction of movement) displayed on the screen.

Consistent with the invention, the method may include capturing a second image of the subject's body part after the subject has moved the body part, as graphically illustrated in Figure 7. "Capturing a second image" may occur in a manner similar to capturing a first image, which is described above in relation to block 704 of Figure 7.

Although not required in its broadest sense, the invention may further include repeating at least portions of method schematically illustrated in Figure 7 until the subject is within a threshold value or the number of repeated attempts reaches a maximum number of re-positioning attempts. For example, after numerous repositioning attempts, the program may instruct the subject to seek technical assistance or may inquire whether the subject wishes to continue the re-positioning effort. Alternatively, the re-positioning may halt when the displacement is within an acceptable range, defined by the threshold value.

Although not required in its broadest sense, the method for facilitating a capture of a body part image may relate to providing a beauty analysis based upon the captured image. The beauty analysis may be similar to the analysis described above in reference to an earlier embodiment.

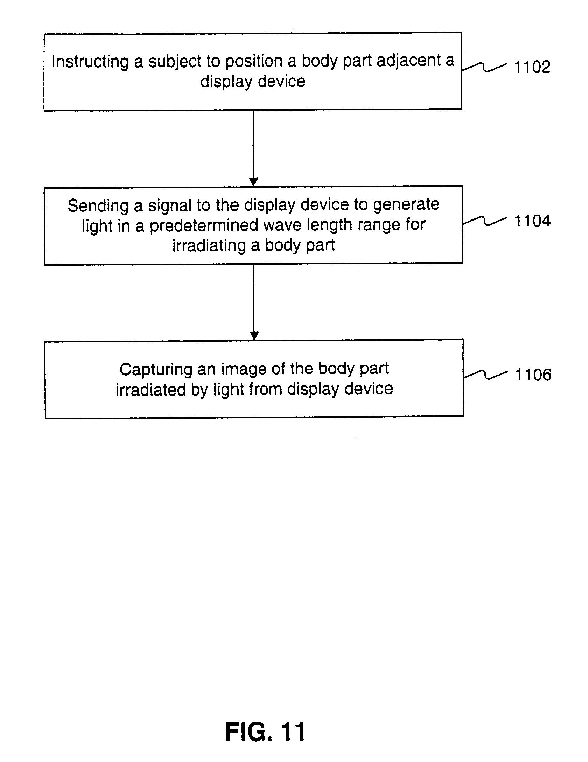

As illustrated in the flow chart of Figure 11, another embodiment of the invention may also include an image capture method. Consistent with the invention, the method may include instructing a subject to position a body part adjacent the display device and within a field of image capture of an image capture device, as graphically illustrated at block 1102 of Figure 11. The phrase "instructing a subject to position a body part" may be performed similar to the instructing of block 304 in Figure 3, described earlier. The instructions may be arranged to encourage the subject to move the body part to a position where the display device might be capable of illuminating the body part sufficiently. In one example, the body part may be the subject's face, but the method could be practiced for other body parts as well.

Consistent with the invention, the method may include sending a signal to the display device to generate light in a predetermined wave length range for irradiating the body part. This is graphically illustrated at block 1104 of Figure 11. The phrase "sending a signal" may involve providing access to at least one of a client-based algorithm and a server-based algorithm. The sending of a signal may be accomplished directly, or indirectly through the provision of software or web access that accomplishes the signal function. In addition, indirect accomplishment of the sending may occur by partnering or affiliating with a third party who sends the signal.

Sending a signal may include transmitting a signal that causes the light generated by the display device to be within a wave length range that effectuates a flash of light from the display device. For example, the display device may serve as a flash for a conventional camera, digital camera or webcam. In this manner, the method may mitigate poor lighting conditions at an image capture location. In addition (or alternatively), the signal might adjust the intensity of the light emitted from the display device to thereby alter the amount of illumination of the body part. In some instances, the signal might cause the display device to darken without generating significant light, for example, to enable multiple images to be obtained at differing levels of light illumination.

The signal may cause the display device to generate light at a wave length within a wave length range that includes red light. By transmitting red light, the display device may provide red eye reduction and/or enable improved visualization of one or more characteristics of the body part.

A method consistent with the invention may also include capturing an image of the body part with the image capture device while the body part is irradiated with the light, in the predetermined wave length range, emitted from the display device. This is illustrated at block 1106 in Figure 11. The process of "capturing" an image may be similar to the process described in an earlier embodiment. For example, the capturing may be performed directly or indirectly as discussed in association with block 306 of Figure 3. The timing of the image capture may be such that it occurs simultaneously while the display device illuminates the body part with light.

Although not required in its broadest sense, the image capture method may be used for beauty analysis and may further include processing the image to derive information about at least one characteristic of the body part. Processing may be performed directly or indirectly in any manner described herein. The processing may include providing access to a client-based algorithm and/or a server- based algorithm. The processing may involve at least partial use of artificial intelligence, such as a neural network, for example. The processing may involve comparing the captured image to a plurality of images maintained in a database. In another example, the method might further include prescribing one or more beauty products to the subject as a function of the characteristic(s) of the body part. A "characteristic" may include skin tone, skin texture, wrinkles, blood vessels, hair, pigmentation, freckles, skin oiliness, and other external body conditions described herein. Prescribing may include transmitting a beauty product recommendation to be sent to the subject or facilitating such transmission through indirect activity. For example, the prescribing could include provided access to a network and/or software providing the product recommendation.

It should be understood that methods in accordance with the invention might include at least portions of differing embodiments described herein. For example, the method graphically illustrated in Figure 11 may be combined with at least portions of the method graphically illustrated in Figure 7, so as to combine features of those methods.

An alternative embodiment of the invention may include a combination. Consistent with the invention, the combination may include at least one tool for gathering information related to beauty. For example, the tool may be configured to gather physical information, physiological information, and/or biological information.

The tool may include one or more of test materials. For example, the test material may be pH indicator, sebutape, a d-squame disc, and a corneodisque indicator. The subject may use a pH indicator, according to electronic or hard-copy instructions, to test a pH value of the skin and/or scalp. Similarly, the subject may use sebutape to remove sebum and other lipids from the surface of the skin or scalp, thereby indicating skin oiliness. A d-squame disc may be used to remove flakes from the surface of the skin or scalp, thereby indicating skin dryness. A corneodisque indicator may leech water from the surface of the epidermis, thereby indicating the moisture/humidity content of the epidermis.

An example of directions for using sebutape and d-squame discs may include instructing the subject to remove the sebutape or d-squame disc from a card, place an index finger behind the tape or disc, place the tape or disc firmly against the forehead, and remove. The directions may instruct the subject to repeat the procedure for the cheek area, using another piece of sebutape. The directions may further specify a time for using the materials. For example, the directions may instruct the subject to wait a designated amount of time after exercising, washing, or applying any beauty products before performing the test. In one embodiment, the directions may instruct the subject to perform the sebutape measurements on a forehead, chin, and cheek area at least five hours after washing the face and applying no moisturizers, make-up or other beauty product to the skin. Directions for the corneodisque indicator and pH indicator may be of a similar nature.

Sebutape, d-squame discs, corneodisque indicators, and pH indicators are inexpensive materials that are easy to use and accurately assess skin type. Other test materials may include ion detectors, mineral detectors, organic detectors, and/or any device capable of measuring a physical, physiological and/or biological parameter. By providing such tests, a vendor may remove the guess work involved in determining a skin type and provide direct visual evidence to the subject for a relatively low cost. Further, the materials may serve as an interactive marketing tool to engage the subject, educate the subject, and improve customer satisfaction.

Other tests of varying cost and scope may also be provided. For example, the test materials may include a device to measure skin elasticity. Similarly, test materials may include hormone tests and enzymatic tests. Test materials may also include various color charts (in hard or soft form), which a subject may use to compare with an external body condition. For example, the subject may use such charts to determine a skin or hair tone, skin or hair color, amount and intensity of wrinkles, acne, balding, hairiness, and/or freckles by selecting the chart that most closely represents an external condition of the subject. Similarly, skin sensitivity may be measured by comparing a pattern of broken blood vessels or capillaries to a chart. The comparison may be completed after applying a particular product to the skin to increase effectiveness. In addition, the test materials may include materials for the subject to compare with a texture of the skin to determine a roughness level of the skin.

. Such test material may be formed at least partially of ceramic material, for example.

Also consistent with the invention, the test materials may include a voucher or other authorization for having one or more tests conducted at a testing facility. The directions for conducting the tests performed by a vendor may include listing available testing facilities and instructing the subject to redeem the voucher at one of the listed testing facilities. As embodied herein, the testing facility may use at least one of a corneometer for measuring a water content of subject's scalp or skin, a cutometer for measuring skin elasticity, a mexameter for measuring a melanin index and hemoglobin (erythema) index of skin, a sebumeter for measuring sebum content of scalp or skin, skin-pH meter for measuring pH of skin or scalp, skin visiometer for measuring skin roughness, sun protection diagnostic to perform an SPF analysis, tewameter to measure trans-epidermal water loss, visioscan to evaluate the skin surface, chromameter to measure skin clarity or luminosity, impedance measurements to measure skin moisture, a gas bearing electrodynamometer to measure skin softness and suppleness, skin replicas or image analysis to measure skin surface smoothness or texture, TEWL to measure skin barrier function, laser doppler to measure blood flow or skin sensitivity, ultrasound to measure skin and fatty tissue thickness, lesion counting and bacteriology to study acne, and ballestometry to measure skin firmness. Hair moisture and suppleness, hair tensile strength, penetration of actives into hair, hair deposition, and UV protection may also be determined. The tests and test materials described above are not intended to be inclusive, it being understood that tests and test materials, as used herein, are intended to cover any method and product for evaluating an external body condition.

Also consistent with the invention, the combination may further include an image capture device for capturing an image of an external body condition of a subject. Consistent with the invention, the combination also may include a driver (e.g., software and/or hardware) for driving the image capture device to capture a external body condition image of a subject, wherein the image capture device, the driver and the at least one tool are packaged and distributed together in order to facilitate an electronic beauty analysis. The image capture device may be a web camera, digital camera, flat bed scanner, ultra-sound image or any other device suitable for image capture.

In an alternative embodiment, the combination may include a driver and at least one tool, without the image capture device. For example, the subject may already have a image capture device. A driver may supplement or enhance the image capture device the subject already has, or the driver may drive a new image capture device. In another embodiment, the combination may include the at least one tool, without an image capture device or a driver for driving the image capture device.

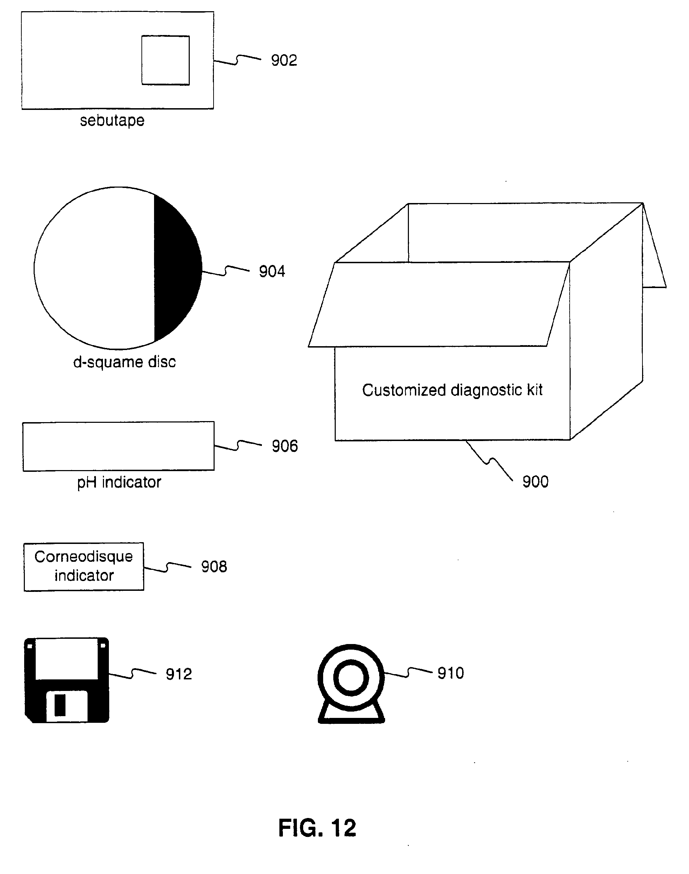

Figure 12 illustrates an example of a combination in the form of a customized diagnostic kit. 900. Based on the subject's answers to personal questions, one or more of sebutape 902, d-squame discs 904, pH indicators 906, and a corneodisque indicator 908 may be selected as a customized set of testing materials for the subject, placed in the kit 900, and delivered to the subject. An image capture device 910 and a driver 912 for the image capture device may also be placed in the kit. The kit 900 may be transmitted to the subject via a courier or other delivery means, or picked up by the subject at a designated location. The customized kit 900 may include material sufficient to conduct a single test or material sufficient to conduct multiple tests.

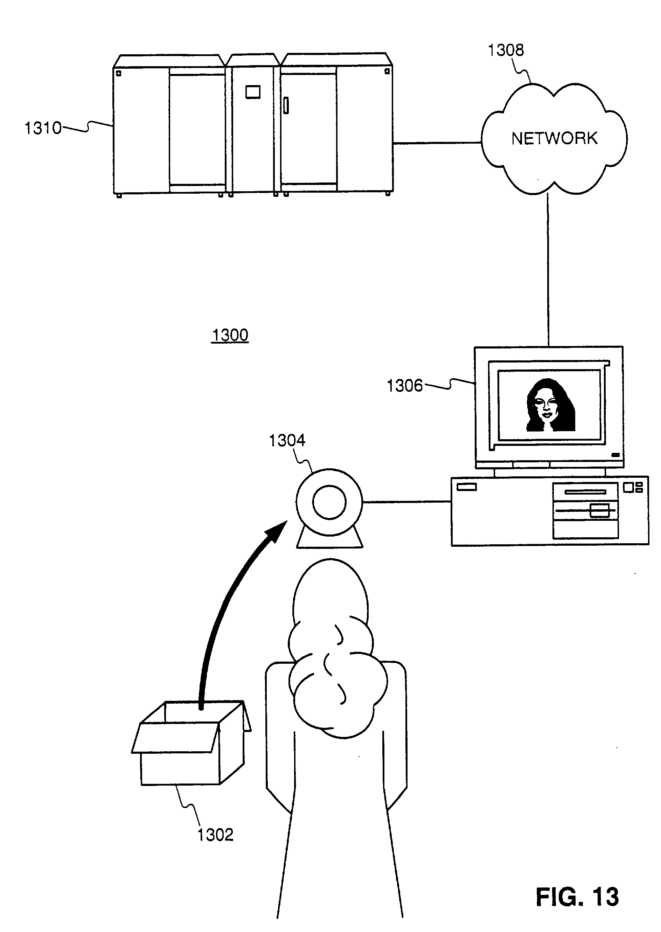

As illustrated in Figure 13, features and principles of the present invention may be implemented in an exemplary system 1300. System 1300 may include a package 1302 containing an image capture device 1304, a computer 1306, network 1308, and mainframe 1310. Components of the system 1300, such as the computer 1306 and/or the mainframe 1308, could be configured and/or loaded with software to perform some or all of one or more of the steps of the methods described herein.

Of course, as a person of ordinary skill in the art will appreciate, system 1300 is one of many systems capable of implementing features and principles of the invention. For example, the methods may be performed wholly by computer 1306 and/or the mainframe 1310, without the network 1308. In another example, the computer 1306 may be a kiosk located in a retail space. Accordingly, it should be appreciated that the system 1300 and the above-mentioned examples are exemplary and not intended to limit the scope of the invention.

Various embodiments herein employ similar terminology. For expert review, the same or similar terms are not necessarily redefined fully with the presentation of each additional embodiment. It is nevertheless to be understood that defined terms and concepts apply equally to all embodiments.

This application may discuss beauty products in connection with use by women. However, it is to be understood that such discussions are for exemplary purposes only. It is to be understood that the invention is equally applicable to all genders, and is not necessarily limited to the beauty industry. It is also to be understood that any functional aspect of the invention can be implemented via any location in the system or network, and data software may be resident at any location either in a network, at a stand-alone site, or on media in the custody and control of a user or subject.

It is to be further understood that the physical mechanisms (e.g. hardware, software, networks, systems) for implementing the methods of the invention are many. Networks, hardware and systems can be configured in a host of ways with software and hardware functionality residing at many alternative locations. In addition, systems other than the exemplary systems disclosed might be used to implement the invention. Therefore, it is to be understood that the methods of the invention are not limited to any particular structure.

Further, methods or portions thereof can be implemented in either an electronic environment, a physical environment, or combinations thereof. Thus, for example, although one or more portions of a method may occur in an electronic environment, a "purchase" portion of the method may occur in a brick and mortar store, or vice versa.

To the extent not inconsistent with the invention defined herein, the following global definitions are to be considered in interpreting the language of this patent and the claims herein. Where multiple definitions are provided, they should be considered as a single cumulative definition.

The term "image" may include one or more of two-dimensional and three-dimensional representations. In certain examples consistent with the invention, a plurality of images from different perspectives may be used to construct a three-dimensional image. In a broader sense, only a single image may be used. Depending on the embodiment, the term "image" may include either a visually perceptible image or electronic image data that may be either used to construct a visually perceptible image or to derive information about the subject. The image may be a body image corresponding to an anatomical portion of the subject, and may represent, for example, the subject's entire face, or a portion of the subject's face. The image may be a detailed picture (e.g., a digital image or a photograph) of a portion of the subject's body and/or a topological plot mapping contours of a portion of subject's body. If the image is representative of an external body condition, the image could be either an actual image showing the condition or an image including symbolizations of the condition, for example. The image may be an actual or a simulated image. Simulated images may include wholly or partially generated computer images, images based on existing images, and images based on stored features of a subject.

The term "image capture device", similar terms, and terms representing structures with similar functions may include one or more of a digital camera, webcam, film camera, analog camera, digital video camera, scanner, facsimile machine, copy machine, infrared imager, ultra-sound imaging device, or any other mechanism for acquiring an image of a subject's external body condition, an image of the subject's countenance, an/or an image of the subject's skin. An ultrasonic device might provide skin thickness information, or it might create a map on an area of the external location. Thus, the term "image" as used herein may be broader than a picture. Combinations of image capture devices may be used. For example, an image captured on photographic paper using a film camera might then be scanned on a flat bed scanner to create another image.

The term "capturing (an image)", or any form thereof, refers to the use of an image capture device to acquire an image. "Capturing" may refer to the direct act of using the image capture device to acquire the image. It may also include indirect acts to promote acquisition. To this end, "capturing" may include the indirect acts of providing access to hardware, or to at least one of a client-based algorithm and a server-based algorithm for causing the image capture device to capture an image. This may be accomplished by providing a user with software to aid in the image capture process, or providing the user with access to a network location at which the software resides. Also consistent with certain embodiments of the invention, capturing may include at least one of receiving an instruction from the subject to capture an image, indicating to the subject before the image is captured, and indicating to the subject when the image is captured.

The term "image processing technique" or similar terms, may include a software program, computer, application specific integrated circuit, electronic device and/or a processor designed to identify in an image one or more characteristics, such as a skin condition. Such techniques may involve binarization, image partitioning, Fourier transforms, fast Fourier transforms (FFTs), and/or discrete cosine transforms may be performed on all or part of the image, resulting in coefficients. Based on the coefficients, conditions may be located, as known in the art. Artificial intelligence, such as fuzzy logic, neural networks, genetic programming and decision tree programming, may also be used to identify conditions. Alternatively, one or more digital filters may be passed through the image for locating specific conditions. These examples are provided for illustrative purposes with the understanding that any image processing technique may be used.

The term "network interface" or similar terms, refer to any mechanism for aiding communications between various nodes or locations in a network. A network interface may include, for example a bus, a modem, or any other input/output structure. A network interface may permit a connection to any network capable of being connected to an input and/or output module located within at least one or more of the following exemplary networks: an Ethernet network, an Internet Protocol network, a telephone network, a radio network, a cellular network, or any mechanism for permitting communication between two or more modes or remote locations. In some invention embodiments, a network interface might also included a user interface.

The term "user interface" may include at least one component such as a keyboard, key pad, mouse, track ball, telephone, scanner, microphone, touch screen, web cam, interactive voice response system (IVR), voice recognition system or any other suitable input mechanism for conveying information. A user interface may also include an input port connected by a wired, optical, or wireless connection for electromagnetic transmissions. In some embodiments, a user interface may include connections to other computer systems to receive the input commands and data therefrom. User interface may further include a data reading device such as a disk drive for receiving input data from and writing data to storage media such as magnetic and optical disks.

As used herein terms such as "external body condition", "skin condition", and "actual condition" refer to conditions of at least one of the skin, teeth, hair, eyebrows, eyelashes, body hair, facial hair, fingernails, and/or toenails, or any other externality. Examples of skin conditions may include elasticity, dryness, cellulitis, sweating, aging, wrinkles, , exfoliation, desquamation, homogeneity of color, creases, liver spots, clarity, lines, micro-circulation, shininess, softness, smoothness, tone, texture, matitty, hydration, sag, suppleness, stress, springiness, firmness, sebum production, cleanliness, translucency, luminosity, irritation, redness, vasocolation, vasomotion, vasodilation, vasoconstriction, pigmentation, freckles, blemishes, oiliness, pore distribution, pore size, moles, birthmarks, acne, blackheads, whiteheads, pockmarks, warts, pustules, boils, blisters, marks, smudges, specks, and other characteristics associated with the subject's skin. Examples of hair conditions may include keratin plug, length, dryness, oiliness, dandruff, pigmentation, thickness, density, root conditions, split ends, hair loss, hair thinning, scales, staging, cleanliness and other properties related to the subject's hair. Examples of fingernail and toenail conditions may include, split nails, delaminating, , brilliancy, lines, spots, coloration, gloss, strength, brittleness, thickness, hangnail, length, , and other characteristics related to the subject's nails. Other conditions may include, for example, size and proportion of facial features, teeth discoloration, and any other aesthetic-related conditions of the user.

"Enabling", "facilitating", and "causing" an action refer to one or more of a direct act of performing the action, and any indirect act of encouraging or being an accessory to the action. Thus, the terms include partnering or cooperating with an entity who performs the action and/or referring commerce to or having commerce referred from an entity who performs the action. Other examples of indirect activity encompassed within the definitions of "enabling", "facilitating", and "causing" may include providing a subject with one or more of tools to knowingly aid in performing the action, providing instructions on how to perform the action, providing prompts or cues to perform the action, or expressly encouraging performance of the action. Indirect activity may also include cooperating with an entity who either directly performs the action or who helps another perform the action. Tools may include software, hardware, or access (either directly, through hyperlink, or some other type of cooperation or partnering) to a network location (e.g., web site) providing tools to aid in performing the action. Thus, phrases such as "enabling access" and "enabling display" do not necessary require that the actor actually access or display anything. For example, the actor may perform the enabling function by affiliating with an entity who performs the action, or by providing instructions, tools, or encouragement for another to do the accessing and displaying.

Forms of the word "displaying" and like terms may also include indirect acts such as providing content for transmission over a network to a display device, regardless of whether the display device is in the custody or control of the sender. Any entity in a chain of delivering information for display performs an act of "displaying", as the term is used herein.

Likewise, the term "providing" includes direct and indirect activities. For example, providing access to a computer program may include at least one of providing access over a network to the computer program, and creating or distributing to the subject a computer program configured to run on the subject's workstation or computer. For example, a first party may direct network traffic to (either through electronic links or through encouragement to visit) a server or web site run by a second party. If the second party maintains a particular piece of software thereon, then it is to be understood that within the meaning of "providing access" as used herein, the first party is said to provide access to the particular software. Or if the first party directs a subject to a second party who in turn ships the particular software to the user, the first party is said to provide the user with access to the particular software. (Of course, in both of the above instances, the second party would also be providing access within the meaning of the phrase as used herein.) "Receiving" may include at least one of acquisition via a network, via verbally communication, via electronic transmission, via telephone transmission, in hard-copy form, or through any other mechanism enabling reception. In addition, "receiving" may occur either directly or indirectly. For example, receipt may occur through a third party acting on another party's behalf, as an agent of another, or in concert with another. Regardless, all such indirect and direct actions are intended to be covered by the term "receiving" as used herein. A received request, for example, may take one of many forms. It may simply be a checked box, clicked button, submitted form or oral affirmation. Or it might be a typed or handwritten textual request. Receiving may occur through an on-line interest form, e-mail, facsimile, telephone, interactive voice response system, or file transfer protocol transmitted electronically over a network at a web site, an internet protocol address, or a network account. A request may be received from a subject for whom information is sought, or an entity acting on the subject's behalf. "Receiving" may involve receipt directly or indirectly through one or more networks and/or storage mediums. Receipt may occur physically such as in hard copy form, via mail delivery or other courier delivery.

Forms of the word "maintain" are used broadly to include gathering, storing, accessing, providing access to, or making something available for access, either directly or indirectly. For example, those who maintain information include entities who provide a link to a site of a third party where the information is stored.

Consistent with the concepts set forth above, all other recited actions such as, for example, obtaining, determining, generating, selecting, applying, simulating, presenting, etc, are inclusive of direct and indirect actions. Thus, for purposes of interpreting the following claims, an entity performs a recited action through either direct or indirect activity. Further examples of indirect activity include sending signals, providing software, providing instructions, cooperating with an entity to have the entity perform the action, outsourcing direct or indirect actions, or serving in any way as an accessory to the specified action.

The term "product" is used to generically refer to tangible merchandise, goods, services, and actions performed. A "beauty product," "beauty care product," "cosmetic product" or similar terms, refer to products (as defined above) for effecting one or more external body conditions, such as conditions of the skin, hair and nails. Examples of tangible merchandise forms of beauty products include cosmetic goods, such as treatment products, personal cleansing products, and makeup products, in any form (e.g., ointments, creams, gels, sprays, supplement, ingesta, inhalants, lotions, cakes, liquids, and powders.)

Examples of services forms of beauty products include hair styling, hair cutting, hair coloring, hair removal, skin treatment, make-up application, and any other offering for aesthetic enhancement. Examples of other actions performed include massages, facial rubs, deep cleansings, applications of beauty product, exercise, therapy, or any other action effecting the external body condition whether performed by a professional, the subject, or an acquaintance of the subject.

The following is exemplary and non-exhaustive listing of a few beauty products- scrubs, rinses, washes, moisturizers, wrinkle removers, exfoliates, toners, cleansers, conditioners, shampoos, cuticle creams, oils, and anti-fungal substances, anti-aging products, anti-wrinkle products, anti-freckle products, skin conditioners, skin toners, skin coloring agents, tanners, bronzers, skin lighteners, hair coloring, hair cleansing, hair styling, elasticity enhancing products, agents, blushes, mascaras, eyeliners, lip liners, lipsticks, lip glosses, eyebrow liners, eye shadows, nail polishes, foundations, concealers, dental whitening products, cellulite reduction products, hair straighteners and curlers, and weight reduction products. A beauty care treatment regimen may involve the administration of one or more products, as defined above.

The terms "beauty advice", "beauty guidance", and similar terms are used interchangeably to refer to the provision of beauty related information to a subject. Advice or guidance includes one or more of beauty product recommendations (e.g., cosmetic product recommendations for products to treat conditions the subject is prompted to evaluate), remedial measures, preventative measures, predictions, prognoses, price and availability information, application and use information, suggestions for complementary products, lifestyle or dietary recommendations, or any other information intended to aid a subject in a course of future conduct, to aid a subject in understanding past occurrences, to reflect information about some future occurrences related to the subject's beauty or to aid a subject in understanding beauty products, as defined above.

The terms "beauty product" and "cosmetic product" refer to a product as defined in the Council Directive 93/35/EEC of 14 June 1993.

The term "network" may include a public network such as the Internet or a telephony network, a private network, a virtual private network, or any other mechanism for enabling communication between two or more nodes or locations. The network may include one or more of wired and wireless connections. Wireless communications may include radio transmission via the airwaves, however, those of ordinary skill in the art will appreciate that various other communication techniques can be used to provide wireless transmission including infrared line of sight, cellular, microwave, satellite, blue-tooth packet radio and spread spectrum radio. Wireless data may include, but is not limited to, paging, text messaging, e-mail, Internet access and other specialized data applications specifically excluding or including voice transmission.

In some instances consistent with the invention, a network may include a courier network (e.g. postal service, United Parcel Service, Federal Express, etc.). Other types of networks that are to be considered within the scope of the invention include local area networks, metropolitan area networks, wide area networks, ad hoc networks, or any mechanism for facilitating communication between two nodes or remote locations.

"Artificial intelligence" (Al) is used herein to broadly describe any computationally intelligent systems that combine knowledge, techniques, and methodologies. An Al engine may be any system configured to apply knowledge and that can adapt itself and learn to do better in changing environments. Thus, the Al engine may employ any one or combination of the following computational techniques: neural network, constraint program, fuzzy logic, classification, conventional artificial intelligence, symbolic manipulation, fuzzy set theory, evolutionary computation, cybernetics, data mining, approximate reasoning, derivative-free optimization, decision trees, or soft computing. Employing any computationally intelligent techniques, the Al engine may learn to adapt to unknown or changing environment for better performance. Al engines may be implemented or provided with a wide variety of components or systems, including one or more of the following: central processing units, co-processors, memories, registers, or other data processing devices and subsystems.

Al engines may be trained based on input such as product information, expert advice, user profile, or data based on sensory perceptions. Using input an Al engine may implement an iterative training process. Training may be based on a wide variety of learning rules or training algorithms. For example, the learning rules may include one or more of the following: back-propagation, real-time recurrent learning, pattern-by-pattern learning, supervised learning, interpolation, weighted sum, reinforced learning, temporal difference learning, unsupervised learning, or recording learning. As a result of the training, Al engine may learn to modify its behavior in response to its environment, and obtain knowledge. Knowledge may represent any information upon which Al engine may determine an appropriate response to new data or situations. Knowledge may represent, for example, relationship information between two or more products. Knowledge may be stored in any form at any convenient location, such as a database.

Since Al engine may learn to modify its behavior, information describing relationships for a universe of all combinations of products may not need to be maintained by the Al engine or any other component of the system.

"Personal information", "subject specific information", "user specific information", "user profile", "personal characteristics", "personal attributes", "profile information", and like terms (collectively referred to in this section as "personal information") may broadly encompass any information about the subject or user. Such information may, for example, fall within categories such as physical characteristics, fashion preferences, demographics, nutritional information, cosmetic usage information, medical history information, environmental information, beauty product usage information, lifestyle, and may include information such as name; age; birth date; height; weight; ethnicity; eating habits; vacation patterns; geographic location of the individual's residence, location, or work; work habits; sleep habits; toiletries used; exercise habits; relaxation habits; beauty care habits; smoking and drinking habits; sun exposure habits; use of sunscreen; propensity to tan; number of sunburns and serious sunburns; dietary restrictions; dietary supplements or vitamins used; diagnosed conditions affecting the external body, such as melanoma; an image, such as a picture or a multimedia file of the subject; facial feature characteristics; family history information such as physical characteristics information about relatives of the subject (e.g., premature balding, graying, wrinkles, etc.); external body condition (as defined previously); color preferences, clothing style preferences, travel habits; entertainment preferences; fitness information; adverse reactions to products, compounds, or elements (e.g., sun exposure); body chemistry, use of prior beauty care products and their effectiveness; purchasing, shopping, and browsing habits; hobbies; marital status; whether the subject is a parent; country of residence; region of residence; birth country and region; religious affiliation; political affiliation; whether the subject is an urban dweller suburban dweller or rural area dweller; size of urban area in which the subject lives; whether the subject is retired; annual income, sexual preference, or any other information reflecting habits, preferences, or affiliations of the subject.

Personal information may also include information electronically gleaned by tracking the subject's electronic browsing or purchasing habits, or as the result of cookies maintained on the subject's computer, responses to surveys, or any other mechanism providing information related to the subject. In addition, personal information may be gathered through non-electronic mechanisms such as hard copy surveys, personal interviews, or consumer preference polls.

"Complementary" and "complementary product" refers to one or more of physical, physiological, biologically, and aesthetic compatibility. A product may be complementary with one or more of another product, a group of products, or a subject. In that latter instance, whether a product is considered "complementary" may be a function of personal information of the subject. Thus, for example a product may be complementary if it is unlikely to cause an adverse allergic reaction; if it physically blends well with another product; or if it is aesthetically consistent with the subject or one or more other products. Aesthetic compatibly may refer to the fact that two products are aesthetically appealing (or do not clash) when worn together. The identification of a complementary product may also be based on product characteristics, user preferences, survey data, or expert advice.

As used herein, the words "may" and "may be" are to be interpreted in an open-ended, non-restrictive manner. At minimum, "may" and "may be" are to be interpreted as definitively including structure or acts recited. Further, the word "or" is to be interpreted in the conjunctive and the disjunctive.

While flow charts presented herein illustrate a series of sequential blocks for exemplary purposes, the order of blocks is not critical to the invention in its broadest sense. Further, blocks may be omitted and others added without departing from the spirit of the invention. Also, the invention may include combinations of features described in connection with differing embodiments.

Although a focus of the disclosure may be on server-side methods, it is nevertheless to be understood that the invention includes corresponding client-side methods, software, articles of manufacture, and computer readable media, and that computer readable media can be used to store instructions for some or all of the methods described herein. Further, it is to be understood that disclosed structures define means for implementing the functionality described herein, and that the invention includes such means for performing the disclosed functions.

In the foregoing Description of Exemplary Embodiments, various features are grouped together in a single embodiment for purposes of streamlining the disclosure. This method of disclosure is not to be interpreted as reflecting an intention that the claimed invention requires more features than are expressly recited in each claim. Rather, as the following claims reflect, inventive aspects lie in less than all features of a single foregoing disclosed embodiment. Thus, the following claims are hereby incorporated into this Description of the Exemplary Embodiments, with each claim standing on its own as a separate embodiment of the invention.

## Claims

1. An image capture method, comprising:
instructing a subject to position a body part adjacent a display device and adjacent an image capture device;
sending a signal to the display device to generate light in a predetermined wave length range for irradiating the body part; and
capturing an image of the body part with the image capture device while the body part is irradiated with the light, in the predetermined wave length range, emitted from the display device.

2. Method according to the preceding claim, wherein the predetermined wave length range includes wave lengths that effectuate a flash of light from the display device.

3. Method according to any one of the two preceding claims, wherein the predetermined wave length includes wave lengths in red light.

4. Method according to any one of the preceding claims, wherein instructing a subject includes at least one of transmitting instructions to the subject over a network, transmitting instructions to the subject in the form of software, and instructing the subject via instructions in hard-copy form.

5. Method according to any one of the preceding claims, wherein sending a signal to the display device includes providing access to at least one of a client-based algorithm and a server-based algorithm for sending the signal.

6. Method according to any one of the preceding claims, enabling beauty analysis wherein the body part is positioned within a field of capture of the image capture device, and further comprising:
processing the image to derive information about at least one characteristic of the body part.

7. Method according to the preceding claim, wherein processing the image includes providing access to at least one of a client-based algorithm and a server-based algorithm.

8. Method according to any one of claims 6 or 7, wherein processing the image is accomplished at least partially using artificial intelligence.

9. Method according to any one of claims 6 to 8, wherein processing the image includes comparing the image to a plurality of images in a database.

10. Method according to any one of claims 6 to 9, further comprising prescribing at least one beauty product to the subject as a function of the at least one characteristic.

11. Method according to any one of claims 6 to 10, wherein the characteristic is chosen from skin tone, skin texture, wrinkles, blood vessels, hair, pigmentation, freckles, and skin oiliness.

12. Method according to any one of the preceding claims, wherein capturing an image includes providing access to at least one of a client-based algorithm and a server-based algorithm for causing the image capture device to capture a calibrating image.

13. Method according to any one of the preceding claims, wherein the body part is a face of the subject.

14. Method according to any one of the preceding claims, further comprising:
capturing a first image of the body part using the image capture device;
processing the first image to thereby calculate at least an approximate first position of the body part relative to at least one of the image capture device and the display device;
comparing the calculated first position with a desired reference position;
instructing the subject to move the body part closer to the desired reference position; and
capturing a second image of the body part after the body part has moved.

15. Method according to the preceding claim, wherein instructing the subject to move includes providing access to at least one of a client-based algorithm and a server-based algorithm for instructing the subject.

16. Method according to any one of the two preceding claims, wherein processing of the first image includes at least one of determining whether the first image includes an entire image of the subject's body part, determining whether the first image includes a portion of the subject's body part, determining whether the subject's body part is centered in the first image, and determining a size of the subject's body part in the first image.

17. Method according to any one of claims 14 to 16, wherein comparing includes calculating a displacement between the calculated first position and the desired reference position, and determining whether the displacement is greater than a threshold value.

18. Method according to the preceding claim, wherein instructing the subject to move includes instructing the subject to move when the displacement is greater than a threshold value.

19. Method according to any one of claims 14 to 18, wherein instructing the subject to move includes providing access to at least one of a client-based algorithm and a server-based algorithm for instructing the subject to move.

20. Method according to any one of claims 14 to 19, wherein capturing a first image of the subject's body part and capturing a second image of the subject's body part include at least one of receiving an instruction from the subject to capture a body part image, indicating to the subject when a body part image is about to be captured, and indicating to the subject when a body part image is captured.

21. Method according to any one of claims 14 to 20, further including:
processing the second image to thereby calculate at least an approximate second position of the subject's body part relative to at least one of the image capture device and the display device;
comparing the calculated second position with a desired reference position to determine a displacement; and
instructing the subject to move the subject's body part closer to the desired reference position when the displacement is greater than a threshold value.

22. An image capture system, comprising:
means for instructing a subject to position a body part adjacent a display device and adjacent an image capture device;
means for sending a signal to the display device to generate light in a predetermined wave length range for irradiating the body part; and
means for capturing an image of the body part with the image capture device while the body part is irradiated with the light, in the predetermined wave length range, emitted from the display device.

23. System according to the preceding claims, wherein the predetermined wave length range includes wave lengths that effectuate a flash of light from the display device.

24. System according to any one of claims 22 or 23, wherein the predetermined wave length includes wave lengths in red light.

25. System according to any one of claims 22 to 24, wherein means for instructing a subject includes at least one of means for transmitting instructions to the subject over a network, means for transmitting instructions to the subject in the form of software, and means for instructing the subject via instructions in hard-copy form.

26. System according to any one of claims 22 to 25, wherein means for sending a signal to the display device includes means for providing access to at least one of a client-based algorithm or server-based algorithm for sending the signal.

27. System according to any one of claims 22 to 26, enabling a beauty analysis, further comprising:
means for instructing a subject to position a body part within a field of capture of the image capture device; and
means for processing the image to derive information about at least one characteristic of the body part.

28. System according to the preceding claim, wherein the means for processing the image includes means for providing access to at least of a client-based algorithm and a server-based algorithm.

29. System according to any one of the two preceding claims, wherein the means for processing the image uses artificial intelligence.

30. System according to any one of claims 27 to 29, wherein the means for processing the image includes means for comparing the image to a plurality of images in a database.

31. System according to any one of claims 27 to 30, further comprising means for prescribing at least one beauty product to the subject as a function of the at least one characteristic.

32. System according to any one of claims 27 to 31, wherein the characteristic is chosen from skin tone, skin texture, wrinkles, blood vessels, hair, pigmentation, freckles, and skin oiliness.

33. System according to any one of claims 27 to 32, wherein the means for capturing an image includes means for providing access to at least one of a client-based algorithm and a server-based algorithm for causing the image capture device to capture a calibrating image.

34. System according to any one of claims 27 to 33, wherein the body part is a face of the subject.

35. System according to any one of claims 27 to 34, further comprising:
means for capturing a first image of the body part using the image capture device;
means for processing the first image to thereby calculate at least an approximate first position of the body part relative to at least one of the image capture device and the display device;
means for comparing the calculated first position with a desired reference position;
means for instructing the subject to move the body part closer to the desired reference position; and
means for capturing a second image of the body part after the body part has moved.

36. System according to the preceding claim, wherein the means for instructing the subject to move includes means for providing access to at least one of a client-based algorithm and a server-based algorithm for instructing the subject.

37. System according to any one of the two preceding claims, wherein the means for processing of the first image includes at least one of means for determining whether the first image includes an entire image of the subject's body part, means for determining whether the first image includes a portion of the subject's body part, means for determining whether the subject's body part is centered in the first image, and means for determining a size of the subject's body part in the first image.

38. System according to any one of claims 35 to 37, wherein the means for comparing includes means for calculating a displacement between the calculated first position and the desired reference position, and means for determining whether the displacement is greater than a threshold value.

39. System according to the preceding claim, wherein the means for instructing the subject to move includes means for instructing the subject to move when the displacement is greater than a threshold value.

40. System according to any one of claims 35 to 39, wherein the means for instructing the subject to move includes means for providing access to at least one of a client-based algorithm and a server-based algorithm for instructing the subject to move.

41. System according to any one of claims 35 to 40, wherein the means for capturing a first image of the subject's body part and the means for capturing a second image of the subject's body part include at least one of means for receiving an instruction from the subject to capture a body part image, means for indicating to the subject when a body part image is about to be captured, and means for indicating to the subject when a body part image is captured.

42. System according to any one of claims 35 to 41, further including:
means for processing the second image to thereby calculate at least an approximate second position of the subject's body part relative to at least one of the image capture device and the display device;
means for comparing the calculated second position with a desired reference position to determine a displacement; and
means for instructing the subject on how to move the subject's body part closer to the desired reference position when the displacement is greater than a threshold value.

43. A combination, comprising:
a least one tool for gathering information related to beauty;
an image capture device for capturing an image of an external body condition of a subject; and
a driver for driving the image capture device to capture the body condition image of the subject, wherein the image capture device, the driver and the at least one tool are packaged and distributed together in order to facilitate an electronic beauty analysis.

44. Combination according to the preceding claim, wherein the tool is configured to gather at least one of physical information, physiological information, and biological information.

45. Combination according to any one of the two preceding claims, wherein the tool comprises at least one of a pH indicator, sebutape, and a corneodisque indicator.

46. A beauty analysis method, comprising:
instructing a subject to position a body part adjacent a display device and within a field of capture of the image capture device;
sending a signal to the display device to generate light in a predetermined wave length range for irradiating the body part;
capturing an image of the body part with the image capture device while the body part is irradiated with the light, in the predetermined wave length range, emitted from the display device; and
processing the image to derive information about at least one characteristic of the body part.

47. A beauty analysis system, comprising:
means for instructing a subject to position a body part adjacent a display device and within a field of capture of an image capture device;
means for sending a signal to the display device to generate light in a predetermined wave length range for irradiating the body part;
means for capturing an image of the body part with the image capture device while the body part is irradiated with the light, in the predetermined wave length range, emitted from the display device; and
means for processing the image to derive information about at least one characteristic of the body part.
